# EUROPÄISCHE PATENTSCHRIFT

(11) **EP 1 019 356 B1**
(45) Veröffentlichungstag und Bekanntmachung des Hinweises auf die Patenterteilung: **27.11.2002**
(21) Anmeldenummer: 98940146.8
(22) Anmeldetag: 06.07.1998
(51) Int. Cl.: C07C 65/00

(54) **VERFAHREN ZUR HERSTELLUNG VON 3-HYDROXY-2-METHYLBENZOESÄURE**
METHOD FOR PRODUCING 3-HYDROXY-2-METHYLBENZOIC ACID
PROCEDE DE PREPARATION D'ACIDE 3-HYDROXY-2-METHYLBENZOIQUE

(30) Priorität: 18.07.1997 DE 19730848
(43) Veröffentlichungstag der Anmeldung: 19.07.2000
(73) Patentinhaber: Bayer Aktiengesellschaft, 51368 Leverkusen (DE)
(72) Erfinder: STELZER, Uwe, D-51399 Burscheid (DE)
(86) Internationale Anmeldenummer: EP9804159
(87) Internationale Veröffentlichungsnummer: WO99003814

(56) Entgegenhaltungen:
- FRESKOS J.N. ET AL.: "(Hydroxyethyl)sulfonamide HIV-1 protease inhibitors: identification of the 2-methylbenzoyl moiety at p-2" BIOORGANIC & MEDICINAL CHEMISTRY LETTERS, Bd. 6, Nr. 4, 1996, Seiten 445-450, XP002089477
- DEAN ET AL: "The Synthesis of Some New Phenols" JOURNAL OF THE CHEMICAL SOCIETY,1961, Seiten 2773-2779, XP002085121

## Beschreibung

Die vorliegende Erfindung betrifft ein neues Verfahren zur Herstellung von 3-Hydroxy-2-methylbenzoesäure.

Es ist bekannt, daß man 3-Hydroxy-2-methylbenzoesäure durch Umsetzung von Natrium-2-naphthylamin-4,8-disulfonat mit 50 %iger NaOH bei 250 bis 260°C erhält. Die Ausbeute beträgt allerdings nur etwa 10 % (J. Chem. Soc. 95, 1883 (1909), siehe auch J. Amer. Chem. Soc. 58, 749 (1936)). Von einer Verbesserung der Ausbeute auf 76 % bei Verwendung eines Anfangsdrucks von 40 bar Stickstoff wird in J. Chem. Soc. 2773 (1961) berichtet. Das nach diesem Verfahren erhaltene Produkt ist jedoch stark verunreinigt, die tatsächliche Ausbeute demnach wesentlich geringer.

Aufgabe der vorliegenden Erfindung war es, ein verbessertes, einfaches und technisch realisierbares Verfahren zur Herstellung von 3-Hydroxy-2-methylbenzoesäure bereitzustellen.

Gegenstand der vorliegenden Erfindung ist ein Verfahren zur Herstellung von 3-Hydroxy-2-methylbenzoesäure der Formel (I) 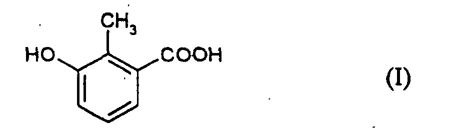 durch Umsetzung von Dinatrium-3-amino-1,5-naphthalindisulfonsäure der Formel (II) 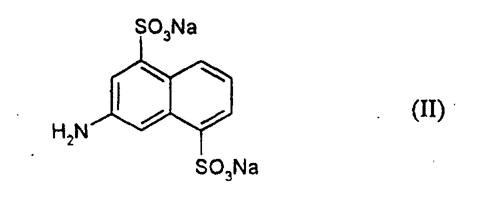 in Gegenwart von Wasser mit 10 bis 20 Äquivalenten Kaliumhydroxid bei Temperaturen von 250 bis 300°C und unter einem Druck von mindestens 100 bar.

Überraschenderweise liefert das erfindungsgemäße Verfahren 3-Hydroxy-2-methylbenzoesäure in höheren Ausbeuten als die bekannten Verfahren. Darüber hinaus fällt das Produkt in einer so hohen Reinheit an, daß es ohne weitere Reinigung für weitere Umsetzung verwendet werden kann.

Das erfindungsgemäße Verfahren läßt sich durch das folgende Formelschema wiedergeben:

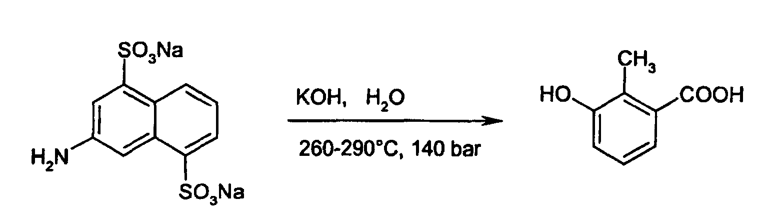

Es werden 10 bis 20 Äquivalente Kaliumhydroxid, bezogen auf die eingesetzte Disulfonsäure der Formel (I) eingesetzt, bevorzugt 14 bis 18, besonders bevorzugt 15 Äquivalente.

Die eingesetzte Wassermenge beträgt im allgemeinen weniger als 55 Gew.-% bezogen auf die KOH-Menge, bevorzugt werden 15 bis 50 Gew.-% Wasser eingesetzt.

Die Reaktionstemperatur beträgt 250 bis 300°C, vorzugsweise 260 bis 290°C.

Der der Reaktionsdruck beträgt mindestens 100 bar, bevorzugt liegt er zwischen 100 und 160 bar.

Im allgemeinen geht man bei der Durchführung des erfindungsgemäßen Verfahrens so vor, daß man die Verbindung der Formel (I) im Autoklaven mit KOH und Wasser vorlegt und mit Stickstoff den gewünschten Druck (beispielsweise 40 bar) einstellt. Dann wird auf die Reaktionstemperatur erhitzt, wodurch sich ein Reaktionsdruck von vorzugsweise mindestens 100 bar einstellt.

Bei der Aufarbeitung geht man im allgemeinen so vor, daß man das Reaktionsgemisch mit Wasser aus dem Autoklaven spült und mit einer Säure (bevorzugt eine anorganische Säure wie Chlorwasserstoffsäure) einen etwa neutralen pH-Wert einstellt.

Das Gemisch wird dann mit Montmorillonit, Kieselgur (beispielsweise (Celite®), Aktivkohle (zum Beispiel Norite®), Bleicherde (zum Beispiel Tonsil®) oder Knochenkohle versetzt, gerührt und filtriert. Dann wird mit einem organischen Lösungsmittel (zum Beispiel Essigsäuremethyl- oder ethylester, Methylenchlorid oder Diethylether) extrahiert und die wässrige Phase mit einer Säure (vgl. oben) auf einen pH-Wert zwischen 1 und 4, bevorzugt 2 und 3 gebracht. Dann wird erneut extrahiert (vgl. oben) und aus den vereinigten organischen Phasen das Reaktionsprodukt in üblicher Weise isoliert (vergleiche auch das Durchführungsbeispiel).

3-Hydroxy-2-methylbenzoesäure ist ein wichtiges Zwischenprodukt für die Synthese insektizider Wirkstoffe (siehe z.B. EP-A-0 602 794, EP-A-0 639 559).

### Beispiel 1

160 g (0,33 mol, 71 %) 3-Amino-1,5-naphthalindisulfonsäuredinatriumsalz werden mit 250 ml H₂O und 330 g KOH (5 mol, 85 %) im Autoklav vorgelegt und mit N₂ ein Druck von 40 bar eingestellt. Man erhitzt 8 Stunden auf 280°C. Der Druck steigt auf 143 bar. Nach dem Abkühlen und Entspannen des Autoklavs wird die schwarzbraune Suspension mit Wasser aus dem Autoklav gespült. Mit konzentrierter HCl wird unter Kühlung auf pH 7,2 gestellt und mit Celite® 15 bis 20 g versetzt, gerührt und filtriert. Die wäßrige Phase wird mit Essigester (2 x 150 ml) extrahiert und anschließend mit konzentrierter HCl auf pH 2 bis 3 gestellt. Hier wird erneut mit Essigester (3 x 150 ml) extrahiert, die vereinigten organischen Phasen getrocknet und das Lösungsmittel im Vakuum entfernt. Man erhält 40,9 g Produkt, welches nach GC 81,6 % der gewünschten, nahezu farblosen 3-Hydroxy-2-methylbenzoesäure enthält. Dies entspricht einer Ausbeute von 66,5 % der Theorie. Schmelzpunkt: 142 bis 143°C.

### Beispiel 2 (Vergleichsbeispiel, vgl. J. Chem. Soc. 2773 (1961))

164 g (0,33 mol, 70 % Dinatrium-3-amino-1,5-naphthalindisulfonsäure werden mit 200 ml H₂O und 200 g NaOH und einem N₂-Anfangsdruck von 40 bar 12 Stunden auf 280°C im Autoklav erhitzt. Der nach Ammoniak riechende Rückstand wird in 1,5 Liter Eiswasser gelöst und unter Kühlung mit konzentrierter HCl angesäuert. Man beobachtet hierbei ein Aufschäumen. Die wäßrige Phase wird mit Essigsäureethylester mehrmals extrahiert, die vereinigten organischen Phasen getrocknet und im Vakuum das Lösungsmittel entfernt. Man isoliert 34,7 g eines grauen Feststoffs, welcher nach GC 77,6% der gewünschten 3-Hydroxy-2-methylbenzoesäure enthält. Hieraus berechnet sich die Ausbeute zu 54 % der Theorie.

## Patentansprüche

1. Verfahren zur Herstellung von 3-Hydroxy-2-methylbenzoesäure der Formel (I) 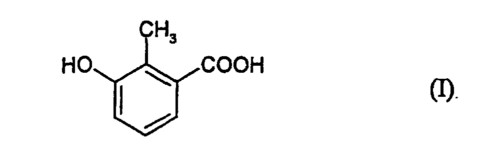 durch Umsetzung von Dinatrium-3-amino-1,5-naphthalindisulfonsäure der Formel (II) 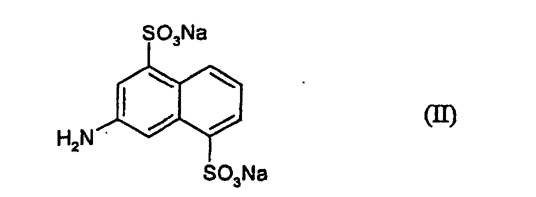 in Gegenwart von Wasser mit 10 bis 20 Äquivalenten Kaliumhydroxid bei Temperaturen von 250 bis 300°C und unter einem Druck von mindestens 100 bar.

## Claims

1. A process for preparing 3-hydroxy-2-methylbenzoic acid of the formula (I) 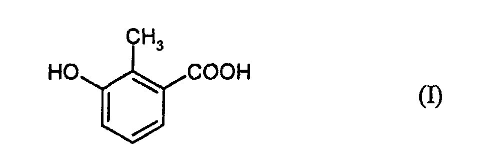 which comprises reacting disodium 3-amino-1,5-naphthalenedisulfonic acid of the formula (II) 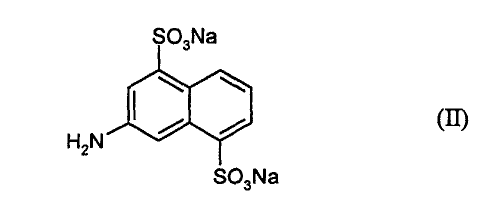 with 10 to 20 equivalents of potassium hydroxide in the presence of water at temperatures from 250 to 300°C and under a pressure of at least 100 bar.

## Revendications

1. Procédé pour la préparation de l'acide 3-hydroxy-2-méthylbenzoïque répondant à la formule (I) 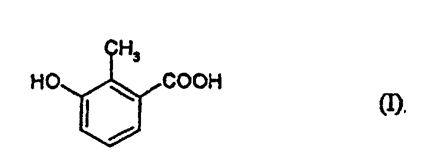 par mise en réaction de l'acide 3-amino-1,5-naphtalènedisulfonique disodique répondant à la formule (II) 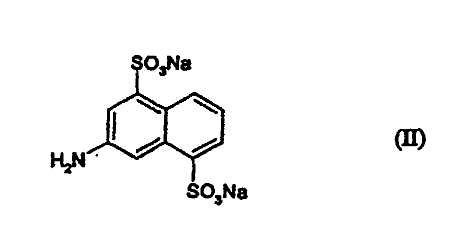 en présence d'eau, avec de 10 à 20 équivalents d'hydroxyde de potassium, à des températures de 250 à 300 °C et sous une pression d'au moins 100 bars.
